# EUROPÄISCHE PATENTSCHRIFT

(11) **EP 3 102 257 B1**
(45) Veröffentlichungstag und Bekanntmachung des Hinweises auf die Patenterteilung: **27.03.2019**
(21) Anmeldenummer: 15702374.8
(22) Anmeldetag: 03.02.2015
(51) Int. Cl.: A61M 5/24, A61M 5/315

(54) **INJEKTIONSGERÄT**
INJECTION DEVICE
DISPOSITIF D'INJECTION

(30) Priorität: 05.02.2014 DE 202014001135 U
(43) Veröffentlichungstag der Anmeldung: 14.12.2016
(73) Patentinhaber: Haselmeier AG, 9001 St. Gallen (CH)
(72) Erfinder: KEITEL, Joachim, 73728 Esslingen (DE)
(74) Vertreter: Reinhardt, Annette
(86) Internationale Anmeldenummer: PCT/EP2015/000206
(87) Internationale Veröffentlichungsnummer: WO 2015/117747

(56) Entgegenhaltungen:
- WO-A1-2013/117332
- DE-A1- 3 638 984
- US-A1- 2009 048 561

## Beschreibung

Die Erfindung betrifft ein Injektionsgerät der im Oberbegriff des Anspruchs 1 angegebenen Gattung.

Aus der EP 1 610 848 B2 ist ein Injektionsgerät bekannt, das als Einstellteil ein Skalenrohr besitzt. Beim Einstellen einer auszupressenden Menge an Injektionsflüssigkeit bewegt sich das Skalenrohr in distaler Richtung. Beim Auspressen der auszupressenden Menge an Injektionsflüssigkeit bewegt sich das Skalenrohr in Gegenrichtung. Das Skalenrohr ist über eine Gewindeverbindung mit dem Gehäuse verbunden, so dass sich das Skalenrohr zusätzlich zur Bewegung in distaler oder proximaler Richtung auch gegenüber dem Gehäuse dreht. Das Injektionsgerät besitzt außerdem eine Rasteinrichtung, die zwischen einem Gewindeteil und dem Gehäuse wirkt. Beim Einstellen der auszupressenden Menge an Injektionsflüssigkeit dreht sich das Gewindeteil gegenüber dem Gehäuse. Beim Auspressen der auszupressenden Menge an Injektionsflüssigkeit ist das Gewindeteil im Gehäuse in axialer Richtung geführt, so dass die Rasteinrichtung beim Auspressen einer Dosis nicht wirksam ist und keine Klicks der Rasteinrichtung zu hören sind.

Das aus der EP 1 610 848 B2 bekannte Injektionsgerät besitzt feste Dosierschritte. Werden für eine Therapie beispielsweise einzustellende Mengen an Injektionsflüssigkeit von 0,20 ml und 0,25 ml benötigt, so sind bekannte Injektionsgeräte so ausgelegt, dass Dosierschritte von höchstens 0,05 ml einstellbar sind. Dies bedeutet zum einen, dass der Anwender mehrere Rastschritte überwinden muss, bis er die kleinste für die Therapie vorgesehene Dosis erreicht. Zum anderen ist bei einem kleinsten festen Dosierschritt von beispielsweise 0,05 ml die beim Priming-Vorgang zu verwerfende Menge an Injektionsflüssigkeit vergleichsweise groß. Für den Priming-Vorgang wären deshalb deutlich kleinere Dosierschritte wünschenswert. Dies führt allerdings zu einer deutlich erhöhten Anzahl von Raststellungen, die der Bediener beim Einstellen der Dosis überwinden muss.

Aus der WO 2013/117332 A1 geht ein Injektionsgerät mit anderem Aufbau hervor. Das Injektionsgerät besitzt ein Dosierorgan, das gegenüber dem Gehäuse drehbar und axial unverschiebbar gelagert ist. Das Dosierorgan ist über eine Gewindeverbindung mit einer Injektionshülse verbunden, die drehfest und axial verschiebbar mit dem Gehäuse verbunden ist. Beim Einstellen einer Injektionsdosis dreht sich das Dosierorgan. Aufgrund der Gewindeverbindung bewegt sich die Injektionshülse in distaler Richtung, dreht sich jedoch nicht. Beim Auspressen der Injektionsflüssigkeit bewegen sich Injektionshülse und Dosierorgan jeweils in Gegenrichtung.

Aus der US 2009/048561 A1 geht ein Injektionsgerät mit einem Einstellelement hervor, das über eine Gewindeverbindung mit dem Gehäuse verbunden ist. Beim Einstellen einer auszupressenden Menge an Injektionsflüssigkeit dreht sich das Einstellteil und bewegt sich in distaler Richtung. Beim Auspressen von Injektionsflüssigkeit bewegt sich das Einstellteil in Gegenrichtung. Eine Rasteinrichtung, die zwischen dem Einstellteil und dem Gehäuse wirkt, ist nicht vorgesehen.

Der vorliegenden Erfindung liegt die Aufgabe zugrunde, ein Injektionsgerät der gattungsgemäßen Art zu schaffen, das die Anordnung mehrerer Raststellungen in unterschiedlichen Abständen ermöglicht.

Diese Aufgabe wird durch ein Injektionsgerät mit den Merkmalen des Anspruchs 1 gelöst.

Die vorliegende Erfindung sieht vor, dass jeder Raststellung eine eindeutige Drehlage des Einstellteils gegenüber dem Gehäuse zugeordnet ist. Dadurch können die benötigten Raststellungen mit unterschiedlichen Abständen zueinander angeordnet werden. Beispielsweise könnte für die eingangs beispielhaft beschriebene Therapie ein Injektionsgerät vorgesehen werden, das genau drei Raststellungen bei 0,01 ml für den Priming-Vorgang und 0,20 ml und 0,25 ml für die zu injizierenden Dosen bereitstellt. Dadurch wird die Bedienung des Injektionsgeräts erheblich vereinfacht.

Bei dem Injektionsgerät gemäß der EP 1 610 848 B2 verändert sich die relative radiale Lage des Gewindeteils gegenüber dem Skalenrohr bei jeder Injektion. Beim Einstellen einer Dosis drehen sich Skalenrohr und Gewindeteil gegenüber dem Gehäuse. Beim Auspressen der auszupressenden Menge an Injektionsflüssigkeit dreht sich das Skalenrohr zurück, während das Gewindeteil drehfest und axial beweglich im Gehäuse geführt ist. Dadurch ist die Drehlage des Gewindeteils im Gehäuse bei einer vorgegebenen einzustellenden Dosis unbestimmt und kann sich bei jedem Injektionsvorgang ändern. Die vorliegende Erfindung sieht dagegen vor, dass jeder Raststellung eine eindeutige Drehlage des Einstellteils gegenüber dem Gehäuse zugeordnet ist. Dadurch können die Raststellungen in unterschiedlichen Abständen zueinander angeordnet werden. Beispielsweise können Raststellungen, die nicht vorgesehenen Mengen an Injektionsflüssigkeit zugeordnet sind, entfallen.

Ein einfacher Aufbau ergibt sich, wenn mindestens eine Raststellung durch mindestens ein drehfest mit dem Gehäuse verbundenes erstes Rastelement und mindestens ein drehfest mit dem Einstellteil verbundenes, mit dem ersten Rastelement zusammenwirkendes zweites Rastelement definiert ist. Die Rastelemente sind dabei immer drehfest mit Gehäuse bzw. Einstellteil verbunden. Dadurch, dass sich das Einstellteil beim Injektionsvorgang zusammen mit dem zweiten Rastelement zurückdreht, kann auf einfache Weise erreicht werden, dass jeder Raststellung eine eindeutige Drehlage des Einstellteils gegenüber dem Gehäuse zugeordnet ist. Vorteilhaft umfasst die Rasteinrichtung ein Rastteil, das unabhängig vom Einstellteil axial verschiebbar ist. Das Rastteil ist dabei drehfest mit dem Gehäuse verbunden. An dem Rastteil ist mindestens ein erstes Rastelement angeordnet. Mindestens ein erstes und mindestens ein zweites Rastelement definieren in einer ersten axialen Stellung von Rastteil und Einstellteil mindestens eine Raststellung. In mindestens einer zweiten axialen Stellung von Rastteil und Einstellteil sind die Rastelemente unabhängig von der Drehlage des Einstellteils zum Rastteil außer Eingriff. Die erste axiale Stellung und die zweite axiale Stellung sind dabei Relativpositionen von Rastteil und Einstellteil zueinander in axialer Richtung. Die Position von Rastteil und Einstellteil zum Gehäuse kann sich dabei ändern. Dadurch, dass die Rastelemente in der zweiten axialen Stellung von Rastteil und Einstellteil außer Eingriff sind, kann sich das Einstellteil gegenüber dem Rastteil in der zweiten axialen Stellung zurückstellen. Die zweite axiale Stellung ist vorteilhaft dann gegeben, wenn die Injektionsflüssigkeit aus dem Injektionsgerät ausgepresst wird. Dadurch sind beim Auspressen der Injektionsflüssigkeit keine Klicks der Rasteinrichtung zu hören. Die zum Auspressen der Injektionsflüssigkeit benötigte Kraft kann auch bei Raststellungen, die beim Einstellen der auszupressenden Menge an Injektionsflüssigkeit deutlich hörbar und spürbar sind, gering gehalten werden.

Vorteilhaft besitzt das Injektionsgerät eine Feder, die das Rastteil in Richtung auf die erste axiale Stellung vorspannt. Die Feder ist insbesondere eine Druckfeder oder eine Zugfeder, die eine Kraft in Richtung einer Längsmittelachse des Injektionsgeräts auf das Rastteil ausübt. Wirkt keine Kraft entgegen der Feder, so ist die Rasteinrichtung aktiv. Die Rastelemente sind vorteilhaft in axialer Richtung ausgerichtet und wirken in axialer Richtung. Vor Erreichen einer Raststellung wird vorteilhaft mindestens ein Rastelement in Richtung der Längsmittelachse des Injektionsgeräts ausgelenkt.

Vorteilhaft lässt die Rasteinrichtung in jeder Raststellung eine Relativdrehung des Einstellteils gegenüber dem Gehäuse in der ersten Drehrichtung zu und sperrt die Relativdrehung in der zweiten Drehrichtung. Dadurch ist ein Zurückdrehen einer einmal eingestellten Dosis nicht möglich. Es kann jedoch auch vorgesehen sein, dass die Rasteinrichtung so ausgelegt ist, dass Raststellungen vom Bediener in der zweiten Drehrichtung überdrückt werden können. Das Injektionsgerät besitzt insbesondere eine Feder, die zwischen Einstellteil und Gehäuse wirkt und die das Einstellteil in der zweiten Drehrichtung vorspannt. Die Feder stellt das Einstellteil zur nächst kleineren Raststellung zurück, wenn sich das Einstellteil nicht in einer Raststellung befindet. Die Feder ist insbesondere eine Torsionsfeder. Die Rasteinrichtung ist dabei vorteilhaft so ausgelegt, dass die Rasteinrichtung die Relativdrehung des Einstellteils in der zweiten Drehrichtung bei dem von der Feder aufgebrachten Drehmoment sperrt. Es kann jedoch vorgesehen sein, dass die Rasteinrichtung durch Aufbringen eines größeren Drehmoments durch den Bediener in der zweiten Drehrichtung aus einer Raststellung zur nächst kleineren Raststellung verstellt werden kann. Durch entsprechende Auslegung von Rasteinrichtung und Feder kann ein Zurückstellen bei einer versehentlich zu groß eingestellten Dosis ermöglicht werden. Durch die Feder kann verhindert werden, dass das Einstellteil in einer Stellung zwischen Raststellungen stehen bleibt, so dass eine nicht vorgesehene Menge an Injektionsflüssigkeit ausgepresst wird. Vorteilhaft besitzt das Injektionsgerät eine erste Feder, die das Rastteil in Richtung auf die erste axiale Stellung vorspannt und eine zweite Feder, die das Einstellteil in der zweiten Drehrichtung vorspannt. Es kann jedoch auch entweder nur die erste Feder oder nur die zweite Feder vorgesehen sein.

Vorteilhaft besitzt das Injektionsgerät eine Kupplung, die in einer ersten Stellung das Einstellteil drehfest mit einem Bedienelement verbindet und die in einer zweiten Stellung eine Relativdrehung des Einstellteils gegenüber dem Bedienelement zulässt. Vorteilhaft steht die Kupplung beim Einstellen einer auszupressenden Menge an Injektionsflüssigkeit in der ersten Stellung und beim Auspressen der Injektionsflüssigkeit aus dem Injektionsgerät in der zweiten Stellung. Dadurch ist eine Bedienung möglich, bei der das Bedienelement beim Einstellen der auszupressenden Menge an Injektionsflüssigkeit zusammen mit dem Einstellteil gedreht wird und bei der das Bedienelement beim Auspressen der auszupressenden Menge an Injektionsflüssigkeit in proximaler Richtung verschoben wird. Dadurch, dass das Einstellteil, das sich beim Auspressen der Injektionsflüssigkeit gegenüber dem Gehäuse dreht, sich auch gegenüber dem Bedienelement drehen kann, muss sich das Bedienelement beim Auspressen der auszupressenden Menge an Injektionsflüssigkeit nicht drehen. Vorteilhaft ist das Bedienelement beim Auspressen der auszupressenden Menge an Injektionsflüssigkeit mindestens über einen Teil seines Verschiebewegs drehfest gegenüber dem Gehäuse geführt.

Das Verstellen der Kupplung von der ersten in die zweite Stellung erfolgt vorteilhaft durch Verschieben des Bedienelements in proximaler Richtung. Das Rastteil ist vorteilhaft in axialer Richtung derart an das Bedienelement gekoppelt, dass eine Bewegung des Bedienelements in proximaler Richtung eine Bewegung des Rastteils in proximaler Richtung bewirkt. Dadurch ist sichergestellt, dass die Rasteinrichtung dann, wenn sich die Kupplung in der zweiten Stellung befindet, also wenn eine Injektion möglich ist, nicht aktiv ist. Ein einfacher Aufbau ergibt sich, wenn das Rastteil und das Bedienelement axial fest und relativ zueinander drehbar miteinander verbunden sind. Eine Feder, die das Rastteil in axialer Richtung vorspannt, wirkt dadurch auch auf das Bedienelement. Vorteilhaft wirkt die Feder auf das Bedienelement in distaler Richtung, so dass das Bedienelement beim Auslösen einer Injektion entgegen der Kraft der Feder in proximaler Richtung bewegt werden muss.

Eine einfache Gestaltung der Kupplung ergibt sich, wenn die Kupplung eine erste Verzahnung am Einstellteil besitzt, die mit einer zweiten Verzahnung am Bedienelement zusammenwirkt. Da sich die relative Lage des Bedienelements gegenüber dem Einstellteil im Betrieb verändern kann, wenn sich das Bedienelement beim Einstellen der auszupressenden Menge an Injektionsflüssigkeit gegenüber dem Gehäuse dreht, beim Injektionsvorgang jedoch drehfest gegenüber dem Gehäuse geführt ist, muss die Kupplung eine drehfeste Verbindung zwischen Bedienelement und Einstellteil in allen theoretisch möglichen Drehlagen von Bedienelement und Einstellteil zueinander ermöglichen. Dies kann auf einfache Weise durch eine entsprechend fein ausgebildete Verzahnung erreicht werden.

Vorteilhaft ist das Einstellteil über eine erste Gewindeverbindung mit dem Gehäuse verbunden und bewegt sich beim Einstellen einer auszupressenden Menge an Injektionsflüssigkeit zusätzlich zur Drehung in der ersten Drehrichtung in distaler Richtung und beim Auspressen einer eingestellten Menge an Injektionsflüssigkeit zusätzlich zur Drehung in der zweiten Drehrichtung in proximaler Richtung. Bei einer bevorzugten Ausführung entspricht der Weg, um den sich das Einstellteil in distaler Richtung bewegt, dem Weg, den ein Dosierkolben des Injektionsgeräts zum Auspressen der eingestellten Menge an Injektionsflüssigkeit zurücklegen muss. Dies ist insbesondere dann vorteilhaft, wenn zwischen dem Einstellteil und dem Gehäuse eine Feder wirkt, die das Einstellteil in der zweiten Drehrichtung vorspannt. Die Drehung des Einstellteils in der zweiten Drehrichtung, die das Auspressen der eingestellten Menge an Injektionsflüssigkeit bewirkt, wird dabei vorteilhaft von der Feder bewirkt, so dass das Auspressen der eingestellten Menge an Injektionsflüssigkeit nach dem Lösen der Raststellung automatisch erfolgt.

In einer bevorzugten Anwendung befindet sich die Injektionsflüssigkeit in einem im Wesentlichen zylindrischen Behälter aus Glas, der an einer Seite mit einem Stopfen verschlossen ist und auf der anderen Seite mit einer Dichtscheibe. Die Dichtscheibe wird durch eine Bördelkappe an den Behälter gedrückt. Bevor Injektionsflüssigkeit ausgepresst werden kann, muss die Dichtscheibe mit einer Injektionsnadel durchstochen werden. Zum Auspressen von Injektionsflüssigkeit wird der Stopfen mit einem Dosierkolben des Injektionsgeräts um den gewünschten Weg verschoben, wodurch eine entsprechende Menge an Injektionsflüssigkeit durch die Injektionsnadel ausgepresst wird. Das Bedienelement ist vorteilhaft mit einem Zustellteil drehfest verbunden, wobei das Zustellteil über eine zweite Gewindeverbindung mit dem Dosierkolben verbunden ist. Der Dosierkolben ist drehfest gegenüber dem Gehäuse gehalten. Beim Einstellen der auszupressenden Menge an Injektionsflüssigkeit bewegt sich das Zustellteil dadurch über die zweite Gewindeverbindung in distaler Richtung. Da das Zustellteil beim Auspressen der auszupressenden Menge an Injektionsflüssigkeit drehfest mit dem Bedienelement verbunden ist, dreht sich das Zustellteil beim Auspressen der auszupressenden Menge an Injektionsflüssigkeit nicht, sondern bewegt sich lediglich in proximaler Richtung. Dadurch wird auch der Dosierkolben in proximale Richtung geschoben und schiebt den Stopfen der Karpule. Vorteilhaft wirkt das Einstellteil derart auf das Zustellteil, dass eine Bewegung des Einstellteils in proximaler Richtung eine Bewegung des Zustellteils in proximaler Richtung bewirkt. Die erste und die zweite Gewindeverbindung können dabei insbesondere so ausgelegt werden, dass das Einstellteil beim Einstellen der auszupressenden Menge an Injektionsflüssigkeit mindestens denselben Weg in distaler Richtung zurücklegt wie das Zustellteil. Vorzugsweise ist vorgesehen, dass Zustellteil und Einstellteil den etwa gleichen Weg in distaler Richtung zurücklegen.

Ein Ausführungsbeispiel der Erfindung wird im Folgenden anhand der Zeichnung erläutert. Es zeigen:
- Fig. 1: eine Seitenansicht eines Injektionsgeräts vor dem Einstellen einer auszupressenden Menge an Injektionsflüssigkeit,
- Fig. 2: das Injektionsgerät aus Fig. 1 nach dem Einstellen einer auszupressenden Menge an Injektionsflüssigkeit,
- Fig. 3: eine Seitenansicht des Injektionsgeräts aus den Figuren 1 und 2 nach dem Auspressen von Injektionsflüssigkeit, wobei der Halter für die Karpule abgenommen ist,
- Fig. 4: einen Schnitt entlang der Linie IV-IV in Fig. 3,
- Fig. 5: einen Schnitt entlang der Linie IV-IV in Fig. 3 in der Stellung des Injektionsgeräts gemäß Fig. 1,
- Fig. 6: einen Schnitt entlang der Linie IV-IV in Fig. 3 in der Stellung des Injektionsgeräts gemäß Fig. 2,
- Fig. 7: eine Seitenansicht eines Bedienelements des Injektionsgeräts,
- Fig. 8: einen Schnitt entlang der Linie VIII-VIII in Fig. 7,
- Fig. 9: eine Ansicht von unten auf das Bedienelement in Richtung des Pfeils IX in Fig. 7,
- Fig. 10: eine Seitenansicht eines Zustellteils des Injektionsgeräts,
- Fig. 11: einen Schnitt entlang der Linie XI-XI in Fig. 10,
- Fig. 12: eine Seitenansicht eines Dosierkolbens des Injektionsgeräts,
- Fig. 13: einen Schnitt entlang der Linie XIII-XIII in Fig. 12,
- Fig. 14: eine perspektivische Darstellung eines Einstellteils des Injektionsgeräts,
- Fig. 15: eine Seitenansicht des Einstellteils aus Fig. 14,
- Fig. 16: einen Schnitt entlang der Linie XVI-XVI in Fig. 15,
- Fig. 17: einen Schnitt entlang der Linie XVII-XVII in Fig. 15,
- Fig. 18: einen Schnitt entlang der Linie XVIII-XVIII in Fig. 15,
- Fig. 19: eine Seitenansicht in Richtung des Pfeils XIX in Fig. 15,
- Fig. 20 bis 23: Seitenansichten des Einstellteils aus Fig. 14,
- Fig. 24: eine perspektivische Darstellung eines Rastteils des Injektionsgeräts,
- Fig. 25: eine Seitenansicht des Rastteils aus Fig. 22,
- Fig. 26: einen Schnitt entlang der Linie XXVI-XXVI in Fig. 25,
- Fig. 27: eine Draufsicht auf das Rastteil in Richtung des Pfeils XXVII in Fig. 25,
- Fig. 28: eine Ansicht von unten in Richtung des Pfeils XXVIII in Fig. 25,
- Fig. 29: eine Seitenansicht eines oberen Gehäuseteils des Injektionsgeräts,
- Fig. 30: einen Schnitt entlang der Linie XXX-XXX in Fig. 29.

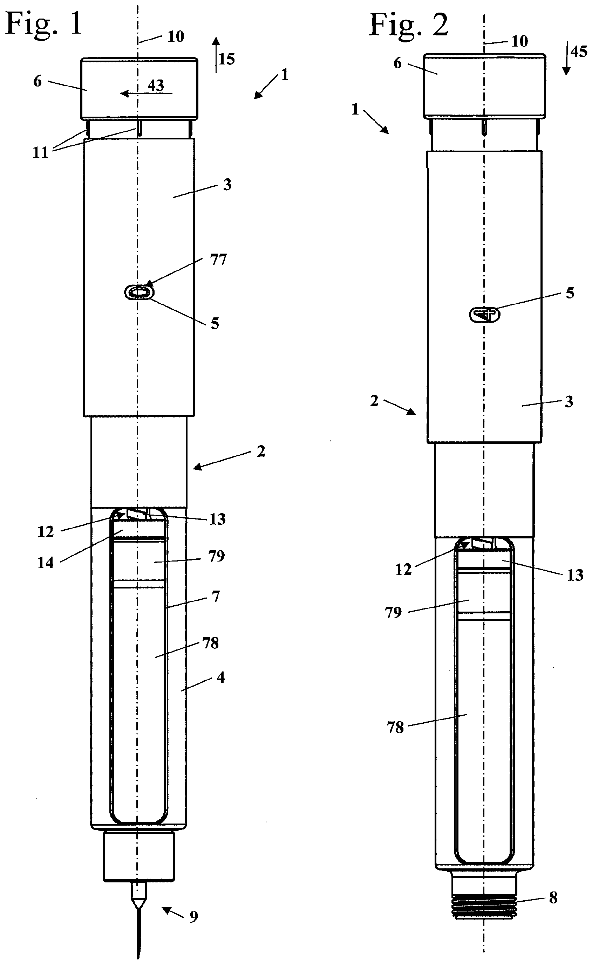

Die Figuren 1 und 2 zeigen ein Injektionsgerät 1, das dazu dient, eine vorgesehene Menge an Injektionsflüssigkeit einzustellen und aus einer in dem Injektionsgerät 1 gehaltenen Karpule auszupressen. Das Injektionsgerät 1 besitzt ein Gehäuse 2, das ein oberes Gehäuseteil 3 sowie einen an dem oberen Gehäuseteil 3 angeordneten Halter 4 umfasst. In dem Halter 4 ist die Karpule angeordnet, die einen vorteilhaft durchsichtigen Behälter 78 mit Injektionsflüssigkeit und einen im Behälter 78 angeordneten, vorteilhaft von außen sichtbaren Stopfen 79 umfasst. Der Halter 4 besitzt im Ausführungsbeispiel zwei einander gegenüberliegend angeordnete Sichtfenster 7, durch die der Bediener sieht, wieviel Injektionsflüssigkeit noch in dem Behälter 78 enthalten ist. An dem Stopfen 79 liegt ein Dosierkolben 12 des Injektionsgeräts 1 an. Der Dosierkolben 12 besitzt eine Kolbenstange 13, an der eine Kolbenscheibe 14 gehalten ist. Die Kolbenscheibe 14 des Dosierkolbens 12 liegt am Stopfen 79 des Behälters 78 an und presst die Injektionsflüssigkeit durch Verschieben des Stopfens 79 in proximaler Richtung aus. Die proximale Richtung bezeichnet dabei die Injektionsrichtung, also die Richtung zu einer Aufnahme für die Injektionsnadel hin bzw. die Richtung, in der die Injektionsflüssigkeit aus dem Behälter 78 ausgepresst wird. Die distale Richtung bezeichnet die entgegengesetzte Richtung, also von der Injektionsnadel weg. Das distale Ende des Injektionsgeräts ist das einer am Injektionsgerät gehaltenen Injektionsnadel abgewandt liegende Ende. Mit "proximal" wird die Seite des Injektionsgeräts bezeichnet, die bei einer Injektion der Einstichstelle zugewandt liegt, und mit "distal" die Seite, die der Einstichstelle abgewandt liegt.

Wie Fig. 1 und Fig. 2 zeigen, besitzt der Halter 4 an seiner proximalen Seite ein Außengewinde 8, auf dem eine Injektionsnadel 9 festgelegt ist. Anstatt des Außengewindes 8 kann auch jede andere Art von Befestigungseinrichtung zur Fixierung der Injektionsnadel 9 am Halter 4 vorgesehen sein. In Fig. 2 ist die Injektionsnadel 9 abgenommen, so dass das Außengewinde 8 sichtbar ist.

An seinem distalen Ende besitzt das Injektionsgerät 1 ein Bedienelement 6. Wie Fig. 1 auch zeigt, sind am Bedienelement 6 an der dem oberen Gehäuseteil 3 zugewandten Seite mehrere Längsrippen 11 angeordnet, die parallel zu einer Längsmittelachse 10 des Injektionsgeräts 1 verlaufen. Das obere Gehäuseteil 3 weist außerdem eine Aussparung 5 auf, durch die eine Skala 77 sichtbar ist, die die eingestellte Menge an Injektionsflüssigkeit anzeigt. Bei der in Fig. 1 gezeigten Stellung des Injektionsgeräts 1 ist keine Dosis eingestellt. Zum Einstellen einer Dosis muss das Bedienelement 6 gegenüber dem Gehäuse 2 in einer ersten Drehrichtung 43, die im Ausführungsbeispiel dem Uhrzeigersinn entspricht, gedreht werden. Dabei bewegt sich das Bedienelement 6 in distaler Richtung, also in Richtung des Pfeils 15 gegenüber dem Gehäuse 2.

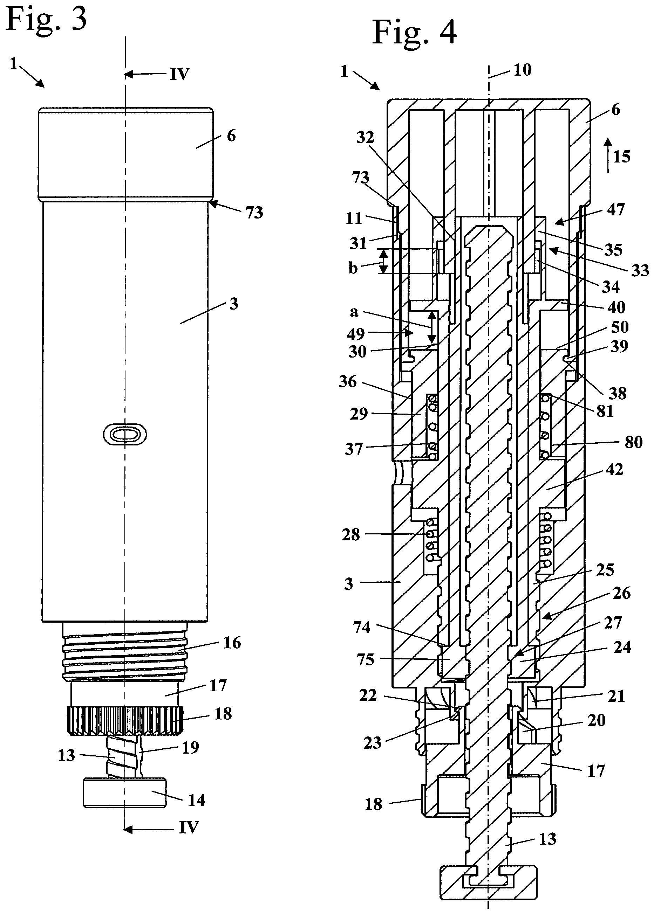

Bei der in Fig. 2 gezeigten Stellung ist eine Dosis eingestellt, die im Ausführungsbeispiel mit der Ziffer "4" bezeichnet ist. Um die eingestellte Dosis auszupressen, muss das Bedienelement 6 in proximaler Richtung, also in Richtung des Pfeils 45 in Fig. 2 gegenüber dem Gehäuse 2 bewegt werden. Dabei wird das Bedienelement 6 entgegen der Kraft einer im Folgenden noch näher beschriebenen Feder 37 (Fig. 4) vom Bediener in proximale Richtung gedrückt. Fig. 3 zeigt das Injektionsgerät 1 in der Stellung, in der das Bedienelement 6 bis zu einem zwischen dem Bedienelement 6 und dem oberen Gehäuseteil 3 gebildeten Anschlag 73 in proximaler Richtung verschoben wurde. Wie auch Fig. 4 zeigt, ist in dieser Stellung des Bedienelements 6 das Bedienelement 6 über die Längsrippen 11, die in Längsnuten 31 des oberen Gehäuseteils 3 ragen, drehfest gegenüber dem Gehäuse 2 gehalten. Um erneut eine auszupressende Menge an Injektionsflüssigkeit einstellen zu können, muss das Bedienelement 6 zunächst in Richtung der Längsmittelachse 10 in distaler Richtung bewegt werden, wie durch den Pfeil 15 angedeutet ist, bis die Längsrippen 11 aus den Längsnuten 31 herausgetreten sind und sich das Bedienelement 6 in der in Fig. 1 gezeigten Stellung befindet. Die Bewegung des Bedienelements 6 in distaler Richtung erfolgt aufgrund der in Fig. 4 gezeigten Feder 37. Nachdem das Bedienelement 6 von der Feder 37 in distaler Richtung verschoben wurde, kann erneut eine auszupressende Menge an Injektionsflüssigkeit durch Drehen des Bedienelements 6 in der ersten Drehrichtung 43 eingestellt werden.

Wie die Figuren 3 und 4 zeigen, besitzt das obere Gehäuseteil 3 an seinem proximalen Ende ein Gewinde 16, auf das der Halter 4 aufgeschraubt werden kann. Am proximalen Ende ragt eine Kolbenstangenführung 17 aus dem oberen Gehäuseteil 3, die an ihrem Außenumfang eine Rändelung 18 aufweist. Die Kolbenstangenführung 17 ist drehfest mit der Kolbenstange 13 verbunden. Hierzu besitzt die Kolbenstange 13 mindestens eine Abflachung 19 an einer Längsseite, in die eine entsprechende Abflachung der Kolbenstangenführung 17 in bekannter Weise eingreift. Die Kolbenstangenführung 17 ist von einer nicht gezeigten Feder in die in den Figuren 3 und 4 gezeigte Stellung vorgespannt. Ist ein Behälter 78 in dem Halter 4 eingelegt und wird der Halter 4 auf das Gewinde 16 aufgeschraubt, so wird die Kolbenstangenführung 17 von dem Behälter 78 in distaler Richtung bewegt. Wie Fig. 4 zeigt, besitzt die Kolbenstangenführung 17 mindestens eine Rampe 20, die in der distalen Position der Kolbenstangenführung 17 mit mindestens einer Rampe 21 des oberen Gehäuseteils 3 zusammenwirkt und die Kolbenstangenführung 17 dadurch drehfest mit dem oberen Gehäuseteil 3 verbindet. Bei montiertem Halter 4 ist dadurch die Kolbenstange 13 drehfest mit dem oberen Gehäuseteil 3 verbunden. Wie Fig. 4 auch zeigt, besitzt die Kolbenstangenführung 17 einen Rastrand 22, der mit einem Rastrand 23 des oberen Gehäuseteils 3 zusammenwirkt und dadurch die Kolbenstangenführung 17 entgegen der Kraft der nicht gezeigten Feder in proximaler Richtung am oberen Gehäuseteil 3 hält. Die Kolbenstangenführung 17 dient dazu, dass die Kolbenstange 13 nach dem Wechsel einer Karpule ins obere Gehäuseteil 3 eingeschraubt werden kann. Hierbei kann der Bediener die Kolbenstangenführung 17 an der Rändelung 18 greifen und gegenüber dem Gehäuse 2 drehen. Bei Injektionsgeräten, bei denen kein Wechsel der Karpule vorgesehen ist, kann die Kolbenstange 13 unmittelbar drehfest am oberen Gehäuseteil 3 gehalten sein. In der in Fig. 4 gezeigten proximalen Stellung der Kolbenstangenführung 17 sind die Rampen 20 und 21 außer Eingriff, so dass die Kolbenstangenführung 17 gegenüber dem oberen Gehäuseteil 3 gedreht werden kann.

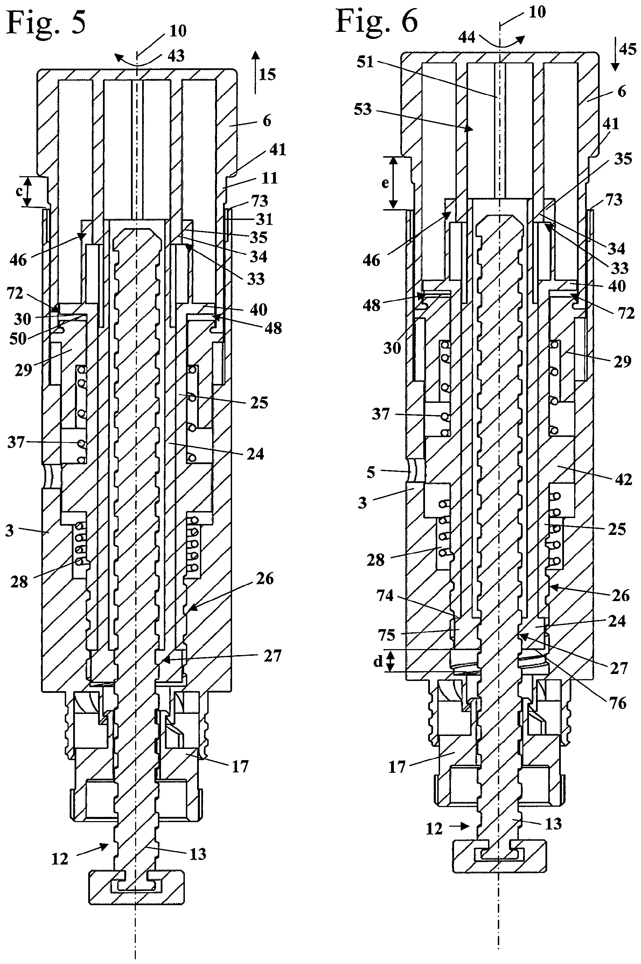

Wie Fig. 4 zeigt, besitzt das Injektionsgerät 1 ein Zustellteil 24, das im Ausführungsbeispiel im Wesentlichen zylindrisch ausgebildet ist und in das die Kolbenstange 13 ragt. Die Kolbenstange 13 ist mit dem Zustellteil 24 über eine Gewindeverbindung 27 verbunden. Eine Drehung des Zustellteils 24 bewirkt aufgrund der drehfesten Fixierung der Kolbenstange 13 im oberen Gehäuseteil 3 eine Bewegung des Zustellteils 24 in distaler Richtung. Das Zustellteil 24 ist über eine Längsführung 32 drehfest mit dem Bedienelement 6 verbunden. Das Injektionsgerät 1 besitzt außerdem ein Einstellteil 25. Das Einstellteil 25 ist im Ausführungsbeispiel ebenfalls im Wesentlichen zylindrisch ausgebildet und am Außenumfang des Zustellteils 24 angeordnet. Das Einstellteil 25 liegt mit einer proximalen Stirnseite 74 an einem Rand 75 des Zustellteils 24 an. Das Einstellteil 25 ist über eine Gewindeverbindung 26 mit dem oberen Gehäuseteil 3 verbunden. Eine Drehung des Einstellteils 25 in der ersten Drehrichtung 43 (Fig. 1) bewirkt aufgrund der Gewindeverbindung 26 eine Bewegung des Einstellteils 25 in distaler Richtung, und eine Drehung in der entgegengesetzten Drehrichtung 44 (Fig. 6) bewirkt eine Bewegung des Einstellteils 25 in proximaler Richtung.

Das Einstellteil 25 besitzt einen Steg 42, der von dem zylindrischen Abschnitt des Einstellteils 25 radial nach außen ragt und an dem eine Feder 28 gehalten ist. Die Feder 28 ist als Torsionsfeder ausgebildet und mit ihrem anderen Ende am oberen Gehäuseteil 3 festgelegt. Die Feder 28 spannt das Einstellteil 25 gegenüber dem oberen Gehäuseteil 3 entgegen der ersten Drehrichtung 43 (Fig. 1) vor.

Zwischen dem Bedienelement 6 und dem Einstellteil 25 wirkt eine Kupplung 33, die sich in Fig. 4 in einer Stellung 47 befindet, in der die Kupplung 33 offen ist. In der Stellung 47 lässt die Kupplung 33 eine Relativdrehung des Einstellteils 25 gegenüber dem Bedienelement 6 zu. Die Kupplung 33 umfasst eine Verzahnung 34 am Bedienelement 6, die in einer Stellung 46 der Kupplung 33 (Fig. 5) in eine Verzahnung 35 am Einstellteil 25 eingreifen und Bedienelement 6 und Einstellteil 25 dadurch drehfest miteinander verbinden kann. Wie Fig. 4 zeigt, besitzt die Verzahnung 34 eine parallel zur Längsmittelachse 10 gemessene Höhe b. In der in den Figuren 3 und 4 gezeigten Stellung liegt das Bedienelement 6 an dem Anschlag 73 des oberen Gehäuseteils 3 auf.

Wie Fig. 4 auch zeigt, ist das Bedienelement 6 in axialer Richtung fest mit einem Rastteil 29 verbunden. Das Rastteil 29 ist hülsenförmig ausgebildet und über eine Längsführung 36 drehfest mit dem oberen Gehäuseteil 3 verbunden. Das Rastteil 29 ist von der Feder 37, die im Ausführungsbeispiel als Druckfeder ausgebildet ist, in distaler Richtung vorgespannt. Das Rastteil 29 besitzt eine Vertiefung 80, in der die Feder 37 angeordnet ist. Die Feder 37 stützt sich an einem Absatz 81 des Rastteils 29 ab. Die Vertiefung 80 begrenzt einen zwischen dem Rastteil 29 und dem Einstellteil 25 ausgebildeten hohlzylindrischen Aufnahmeraum für die Feder 37. Zur axial festen Verbindung des Bedienelements 6 mit dem Rastteil 29 dient ein Rastrand 39 des Bedienelements 6, der in eine Vertiefung 38 des Rastteils 29 ragt. Es kann jedoch auch vorgesehen sein, dass das Bedienelement 6 nur bei einer Bewegung in proximaler Richtung auf das Rastteil 29 wirkt und eine dem Bedienelement 6 folgende Bewegung des Rastteils 29 in distaler Richtung aufgrund der Feder 37 erfolgt.

Das Rastteil 29 besitzt eine Stirnseite 50, die einem Rand 40 des Einstellteils 25 zugewandt liegt. Die Stirnseite 50 ist die distale Stirnseite des Rastteils 29. An der Stirnseite 50 ist ein erstes Rastelement 30 des Rastteils 29 angeordnet. Das erste Rastelement 30 besitzt in der in Fig. 4 gezeigten Stellung zum Rand 40 einen in Richtung der Längsmittelachse 10 gemessenen Abstand a. Das Rastelement 30 ist außer Eingriff mit einem in Fig. 4 nicht gezeigten, an der der Stirnseite 50 zugewandten Seite des Rands 40 angeordneten Rastelement am Einstellteil 25. Einstellteil 25 und Rastteil 29 befinden sich in Fig. 4 in einer zweiten axialen Stellung 49, in der unabhängig von der Drehlage von Einstellteil 25 und Rastteil 29 keine Verrastung zwischen Einstellteil 25 und Rastteil 29 möglich ist.

Nach dem Auspressen von Injektionsflüssigkeit wird das Bedienelement 6 von der Feder 37 aus der in Fig. 4 gezeigten Stellung in Richtung des Pfeils 15, also in distaler Richtung, bewegt, bis sich das Bedienelement 6 in der in Fig. 5 gezeigten Stellung befindet. Das Bedienelement 6 besitzt einen Absatz 41, der in Fig. 4 am Anschlag 73 anliegt und in Fig. 5 zum Anschlag 73 einen Abstand c besitzt. Der Abstand c entspricht dem in Fig. 4 gezeigten Abstand a zwischen dem Rastelement 30 und dem Rand 40. Fig. 5 zeigt die zwischen dem Einstellteil 25 und dem Rastteil 29 gebildete Rasteinrichtung 72 in einer ersten axialen Stellung 48, in der das erste Rastelement 30 mit einem am Einstellteil 25 angeordneten Rastelement zusammenwirken und Raststellungen des Injektionsgeräts 1 definieren kann. Das Rastteil 29 liegt in der ersten axialen Stellung 48 mit seinem Rastelement 30 am Rand 40 an. Wie Fig. 5 auch zeigt, sind die Längsrippen 11 am Bedienelement 6 kürzer als der Abstand c, so dass die Längsrippen 11 in der in Fig. 5 gezeigten Stellung des Injektionsgeräts 1 aus dem Bereich der Längsnuten 31 gelangt sind. Bei der Bewegung des Bedienelements 6 in proximaler Richtung hat sich die Verzahnung 34 des Bedienelements 6 in die Verzahnung 35 des Einstellteils 25 geschoben. In der in Fig. 5 gezeigten ersten Stellung 46 der Kupplung 33 sind Bedienelement 6 und Einstellteil 25 drehfest miteinander verbunden.

Zum Einstellen einer Injektionsdosis muss das Bedienelement 6 in der ersten Drehrichtung 43 um die Längsmittelachse 10 gedreht werden. Aufgrund der drehfesten Verbindung zwischen Bedienelement 6 und Zustellteil 24 dreht sich auch das Zustellteil 24. Über die zweite Gewindeverbindung 27 bewegt sich das Zustellteil 24 zusätzlich in distaler Richtung. Das Einstellteil 25 ist über die Kupplung 33 ebenfalls drehfest mit dem Bedienelement 6 verbunden und bewegt sich bei seiner Drehung aufgrund der Gewindeverbindung 26 ebenfalls zusätzlich in distaler Richtung. Dabei kann der Weg, den das Zustellteil 24 und das Einstellteil 25 zurücklegen, etwa gleich groß sein. Vorzugsweise ist der Weg, den das Einstellteil 25 zurücklegt, etwas größer als der Weg des Zustellteils 24, so dass eine Bewegung des Dosierkolbens 12 in proximaler Richtung beim Einstellen einer Dosis vermieden werden kann. Das Rastteil 29 wird von der Feder 37 in distaler Richtung bewegt und der Bewegung des Einstellteils 25 nachgeführt. Dadurch bewegt sich auch das fest mit dem Rastteil 29 verbundene Bedienelement 6 in distaler Richtung.

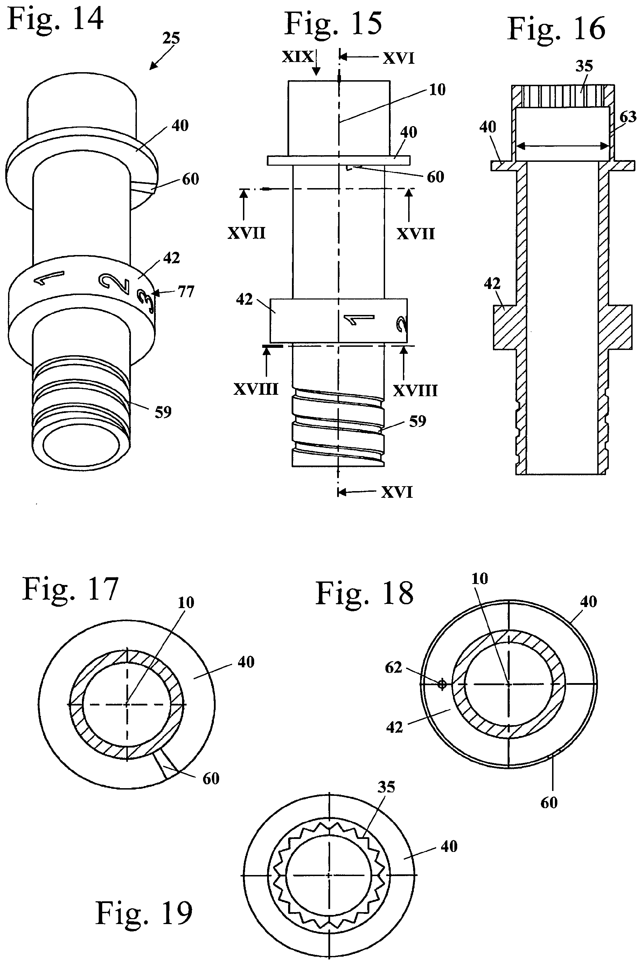

Fig. 6 zeigt das Injektionsgerät 1 nach dem Einstellen einer auszupressenden Menge an Injektionsflüssigkeit. Der Absatz 41 besitzt zum Anschlag 73 einen Abstand e, der deutlich größer als der Abstand c ist. Das Zustellteil 24 besitzt eine proximale Stirnseite 76, die sich gegenüber der in Fig. 5 gezeigten Stellung um einen Weg d in distaler Richtung bewegt hat. Der Abstand e entspricht der Summe des Wegs d und des in Fig. 5 gezeigten Abstands c. Durch die Aussparung 5 des Gehäuseteils 3 ist der Steg 42 des Einstellteils 25 sichtbar, an dessen Außenumfang die Skala 77 angeordnet ist (Fig. 14).

Zum Auspressen einer eingestellten Menge an Injektionsflüssigkeit bewegt der Bediener das Bedienelement 6 entgegen der Kraft der Feder 37 in proximaler Richtung, wie durch den Pfeil 45 angedeutet ist. Dadurch bewegt sich das Bedienelement 6 relativ zum Einstellteil 25. Die Kupplung 33 wird in die in Fig. 4 gezeigte zweite Stellung 47 verstellt. Auch das Rastelement 30 bewegt sich in proximaler Richtung gegenüber dem Einstellteil 25, so dass das erste Rastelement 30 außer Eingriff mit einem am Rand 40 angeordneten Rastelement gelangt. Die Rasteinrichtung 72 wird durch die Bewegung des Bedienelements 6 in proximaler Richtung in die zweite axiale Stellung 49 verstellt, die in Fig. 4 gezeigt ist. Im Ausführungsbeispiel wird zuerst die Rasteinrichtung 72 außer Eingriff gebracht, und anschließend wird die Kupplung 33 gelöst. Das Einstellteil 25 wird, sobald die Kupplung 33 und die Rasteinrichtung 72 gelöst sind, von der Feder 28 in der zweiten Drehrichtung 44 (Fig. 6) gedreht, die der ersten Drehrichtung 43 entgegengerichtet liegt. Über die Gewindeverbindung 26 bewegt sich das Einstellteil 25 zusätzlich zur Drehung in Richtung des Pfeils 45 in proximaler Richtung. Die Stirnseite 74 des Einstellteils 25 wirkt auf den Rand 75 des Zustellteils 24 und verstellt das Zustellteil 24 in proximaler Richtung um den Weg d. Da das Zustellteil 24 drehfest mit dem Bedienelement 6 verbunden ist, kann sich das Zustellteil 24 nicht drehen. Der drehfest mit dem oberen Gehäuseteil 3 verbundene Dosierkolben 12 bewegt sich deshalb in proximaler Richtung und presst Injektionsflüssigkeit aus dem Behälter aus.

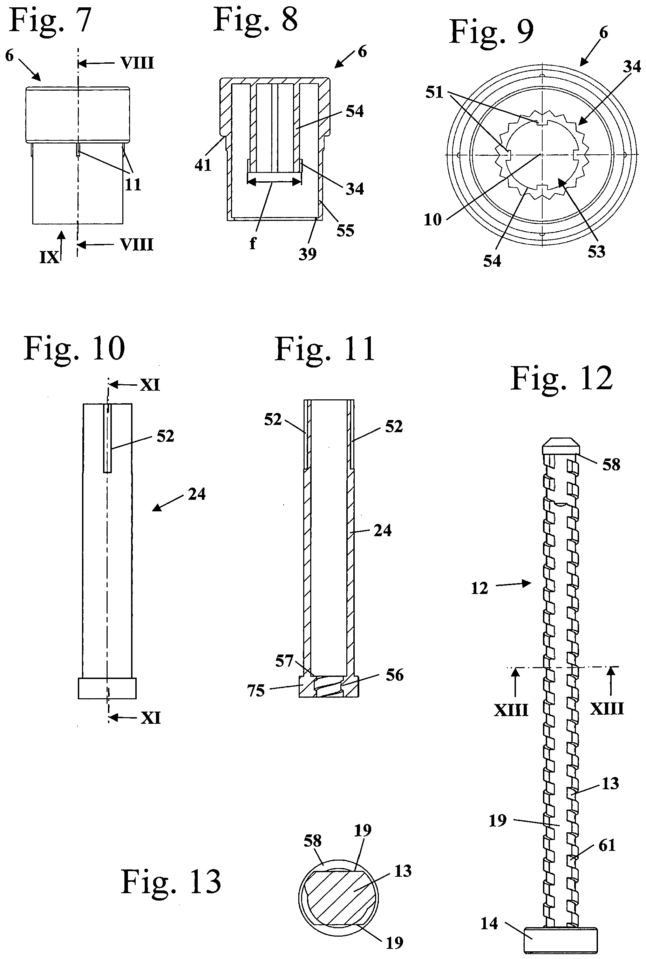

Die Figuren 7 bis 26 zeigen die Teile des Injektionsgeräts 1 im Einzelnen. Die Figuren 7 bis 9 zeigen das Bedienelement 6. In Fig. 7 sind die Längsrippen 11 am Bedienelement 6 gezeigt. Im Ausführungsbeispiel sind vier am Umfang gleichmäßig verteilt angeordnete Längsrippen 11 vorgesehen.

Wie die Figuren 8 und 9 zeigen, ist das Bedienelement 6 etwa zylindrisch ausgebildet und besitzt eine Hülse 55, an deren proximalem Ende der nach innen ragende Rastrand 39 angeordnet ist. An ihrem distalen Ende ist die Hülse 55 verschlossen. Innerhalb der Hülse 55 ist ein etwa zylindrischer Stutzen 54 angebracht, der vollständig innerhalb der Hülse 55 liegt und an dessen proximaler Seite die Verzahnung 34 ausgebildet ist. Im Ausführungsbeispiel ist die Verzahnung 34 an der Außenseite des Stutzens 54 ausgebildet. Die Verzahnung 34 besitzt einen Außendurchmesser f. Wie die Figuren 8 und 9 auch zeigen, sind am Innenumfang des Stutzens 54 Stege 51 vorgesehen, die mit in den Figuren 10 und 11 gezeigten Nuten 52 am Außenumfang des Zustellteils 24 zusammenwirken und das Bedienelement 6 in jeder Relativposition von Bedienelement 6 und Zustellteil 24 drehfest miteinander verbinden. Wie die Figuren 6 und 9 zeigen, begrenzt der Stutzen 54 eine Aufnahme 53, in die das Zustellteil 24 ragt.

Wie Fig. 11 zeigt, besitzt das Zustellteil 24 im Bereich des Rands 75 ein Innengewinde 56. Die Kolbenstange 13 besitzt ein in Fig. 12 gezeigtes Außengewinde 61, das in das Innengewinde 56 des Zustellteils 24 eingeschraubt ist. Am Rand 75 ist ein Absatz 57 angeordnet, an den ein Anschlag 58 der Kolbenstange 13 anschlägt, wenn eine Dosis eingestellt wird, die größer als die in der Karpule vorhandene Restmenge ist. Der Anschlag 58 ist am distalen Ende der Kolbenstange 13 angeordnet und besitzt einen größeren Außendurchmesser als das Außengewinde 61 der Kolbenstange 13. Um eine Montage der Kolbenstange 13 am Zustellteil 24 zu erlauben, ist die Kolbenscheibe 14 von der Kolbenstange 13 separat ausgebildet und an dieser befestigt. Wie Fig. 13 zeigt, besitzt die Kolbenstange 13 zwei an gegenüberliegenden Längsseiten der Kolbenstange 13 angeordnete Abflachungen 19 zur drehfesten Verbindung mit der Kolbenstangenführung 17. Auch eine andere Gestaltung der drehfesten Verbindung kann jedoch vorteilhaft sein.

Die Figuren 14 bis 19 zeigen die Gestaltung des Einstellteils 25 im Einzelnen. Auch das Einstellteil 25 ist etwa hülsenförmig ausgebildet und besitzt den nach außen stehenden Rand 40, an dessen proximaler Seite ein zweites Rastelement 60 angeordnet ist. Das zweite Rastelement 60 wirkt mit Rastelementen am Rastteil 29 zusammen und bildet mit diesen die Rasteinrichtung 72. In Fig. 15 ist das Rastelement 60 schematisch in Seitenansicht gezeigt. Das Rastelement 60 befindet sich in Fig. 15 hinter der Zeichenebene. Das Rastelement 60 ist rampenförmig ausgebildet, wobei die etwa parallel zur Längsmittelachse 10 verlaufende Flanke des Rastelements 60 bei einer Drehung des Einstellteils 25 in der zweiten Drehrichtung 44 vorlaufend und die flach abfallende Flanke bei einer Drehung des Einstellteils 25 in der ersten Drehrichtung 43 vorlaufend angeordnet ist. Im proximalen Bereich des Zustellteils 25 ist ein Außengewinde 59 vorgesehen, das Teil der ersten Gewindeverbindung 26 ist.

Wie die Figuren 16 und 19 zeigen, ist die Verzahnung 35 der Kupplung 33 an der distalen Seite des Einstellteils 25 angeordnet und nach innen gewandt. An die proximale Seite der Verzahnung 35 schließt sich ein hülsenförmiger Abschnitt 63 des Einstellteils 25 an, dessen Innendurchmesser g größer als der in Fig. 8 gezeigte Außendurchmesser f der Verzahnung 34 ist. Sobald sich die Verzahnung 34 im hülsenförmigen Abschnitt 63 befindet, sind die Verzahnungen 34 und 35 außer Eingriff, und das Bedienelement 6 ist gegenüber dem Zustellteil 25 drehbar.

Wie Fig. 17 zeigt, erstreckt sich das zweite Rastelement 60 über die gesamte in radialer Richtung zur Längsmittelachse 10 gemessene Breite des Rands 40. Im Ausführungsbeispiel ist ein einziges zweites Rastelement 60 vorgesehen. Auch eine andere Anzahl zweiter Rastelemente 60 kann jedoch vorteilhaft sein, beispielsweise, wenn nur ein geringer Verdrehwinkel des Bedienelements 6 benötigt und ein deutliches Spüren und Hören der Raststellungen gewünscht wird. Wie Fig. 18 zeigt, besitzt der Steg 42 eine Öffnung 62. In der Öffnung 62 ist die Feder 28 eingehängt.

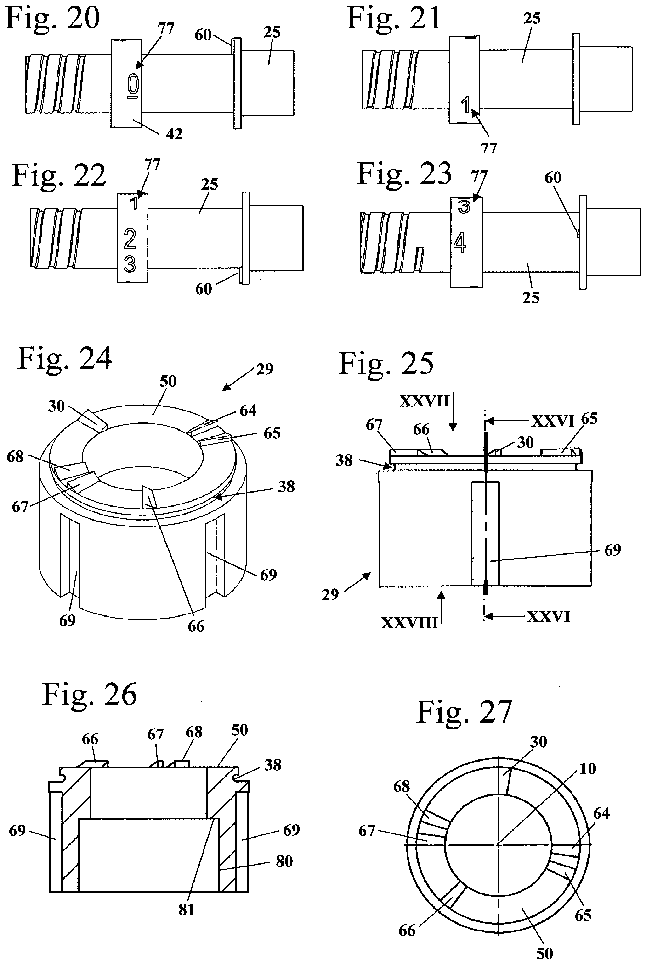

In den Figuren 20 bis 23 ist die auf dem Steg 42 des Einstellteils 25 aufgebrachte Skala 77 im Einzelnen gezeigt. Die einzelnen Werte der Skala 77 besitzen in Umfangsrichtung unterschiedliche Abstände zueinander. Auch der seitliche Versatz zwischen den Werten, also der Versatz in Richtung der Längsmittelachse 10 (Fig. 15) ist unterschiedlich. Die jeweils von der Mitte der Werte der Skala 77 gemessenen Abstände der Werte in Umfangsrichtung entsprechen den Abständen der zugeordneten Rastelemente am Rastteil 29. Der unterschiedliche seitliche Versatz ergibt sich aus dem axialen Weg, den das Einstellteil 25 von einer Raststellung zur nächsten Raststellung zurücklegt.

Die Figuren 24 bis 28 zeigen das Rastteil 29 im Einzelnen. Das Rastteil 29 ist etwa hülsenförmig ausgebildet und besitzt an seiner Stirnseite 50 die ersten Rastelemente 30, 64, 65, 66, 67 und 68. Das Rastelement 64 ist der Nullstellung zugeordnet, und das in kurzem Abstand in Umfangsrichtung zum Rastelement 64 angeordnete erste Rastelement 65 entspricht der Position zum Primen. Die Rastelemente 66, 67, 68 und 30, die in unterschiedlichen Abständen zueinander angeordnet sind, entsprechen unterschiedlichen einzustellenden Mengen an Injektionsflüssigkeit. Alle ersten Rastelemente 30 und 64 bis 68 sind rampenförmig ausgebildet, wobei die bei einer Drehung des Einstellteils 25 in Drehrichtung 43 vorne liegende Flanke leicht angeschrägt ist und die hinten liegende Flanke steil abfällt. Wird das Einstellteil 25 gegenüber dem Rastteil 29 in der ersten Drehrichtung 43 bewegt, so gleitet die flach abfallende Flanke des zweiten Rastelements 60 an einer angeschrägt verlaufenden Flanke eines der Rastelemente 30 und 64 bis 68 ab. Dadurch wird eine Kraft auf das Rastteil 29 in proximaler Richtung erzeugt, die zu einer Auslenkung des Rastteils 29 entgegen der Kraft der Feder 37 führt. Dadurch ist das Erreichen einer Raststellung für den Bediener hör- und spürbar.

Wie die Figuren 24 bis 26 zeigen, besitzt das Rastteil 29 unmittelbar benachbart zur Stirnseite 50 die Vertiefung 38, in die der Rastrand 39 des Bedienelements 6 eingreift. Wie Fig. 26 zeigt, besitzt das Rastteil 29 an der der Stirnseite 50 abgewandten Seite die Vertiefung 80, an der das Rastteil 29 einen vergrößerten Innendurchmesser besitzt. Die Vertiefung 80 geht mit einem Absatz 81 in einen Abschnitt mit geringerem Innendurchmesser über. Die Vertiefung 80 begrenzt gemeinsam mit dem Einstellteil 25 einen Aufnahmeraum für die Feder 37. Am Absatz 81 stützt sich die Feder 37 ab. Wie die Figuren 24, 25, 26 und 28 zeigen, sind vier Vertiefungen 69 gleichmäßig am Umfang des Rastteils 29 verteilt. Die Vertiefungen 69 verlaufen parallel zur Längsmittelachse 10 des Injektionsgeräts 1 und dienen zur drehfesten Verbindung des Rastteils 29 mit dem oberen Gehäuseteil 3. Auch Fig. 27 zeigt die ungleichmäßige Anordnung der ersten Rastelemente an der Stirnseite 50. Die ersten Rastelemente 30 und 64 bis 68 können nahezu beliebig an der Stirnseite 50 angeordnet werden. Vorteilhaft ist das Bedienelement 6 bis zum Erreichen der maximalen Dosis um weniger als eine volle Umdrehung drehbar. Dadurch wird beim Einstellen der Dosis jedes Rastelement höchstens einmal überfahren, so dass eine beliebige Anordnung der Rastelemente und dadurch eine beliebige Wahl einzustellender Mengen an Injektionsflüssigkeit möglich ist.

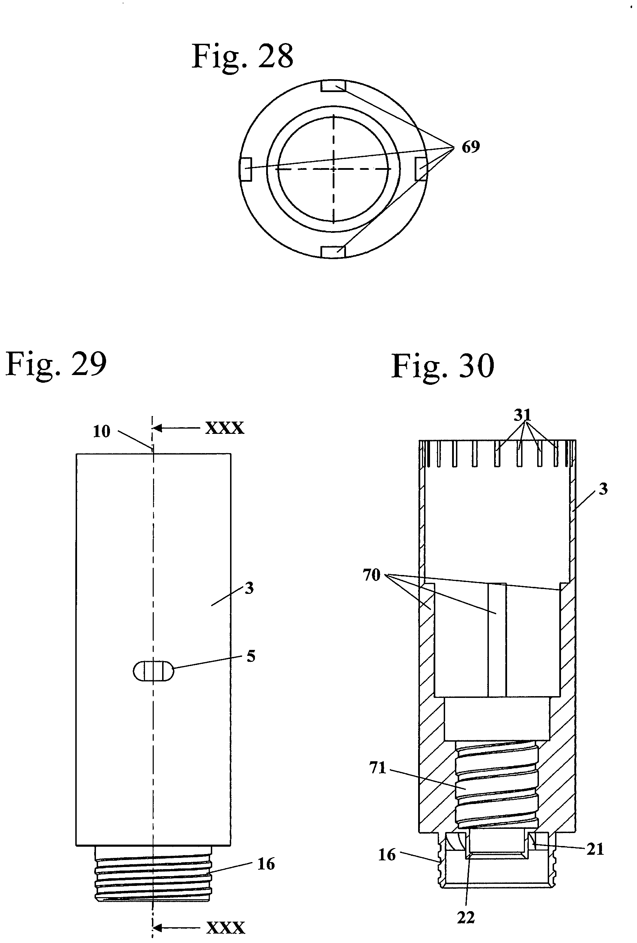

Die Figuren 29 und 30 zeigen das obere Gehäuseteil 3. Wie Fig. 29 zeigt, besitzt das obere Gehäuseteil 3 Längsstege 70 an seiner Innenseite, die in die Vertiefungen 69 des Rastteils 29 eingreifen und dadurch das Rastteil 29 drehfest im Gehäuse 2 halten. Das Gehäuseteil 3 besitzt in seinem proximalen Bereich ein Innengewinde 71, das mit dem Außengewinde 59 des Einstellteils 25 (Fig. 14) zusammenwirkt und mit diesem die erste Gewindeverbindung 26 bildet. Wie Fig. 26 auch zeigt, besitzt das obere Gehäuseteil 3 an seinem distalen Ende eine Vielzahl von Längsnuten 31, so dass das Bedienelement 6 in unterschiedlichen Drehstellungen in das Gehäuseteil 3 gedrückt werden kann.

Dadurch, dass das Einstellteil 25 sich beim Einstellen der auszupressenden Menge an Injektionsflüssigkeit gegenüber dem Gehäuse 2 dreht und sich beim Auspressen der Dosis um den gleichen Weg zurückdreht, befinden sich das Einstellteil 25 und das drehfest am Gehäuse 2 gehaltene Rastteil 29 in jeder Raststellung in einer eindeutig definierten Drehlage zueinander. Da die Drehlage des Einstellteils bei einem Einstellteil, das beim Einstellen einer Injektionsdosis höchstens eine ganze Umdrehung zurücklegt, auch immer einer Injektionsdosis entspricht, kann für jede Dosis eine separate Raststellung vorgesehen werden. Zwischenrastschritte werden nicht benötigt.

## Patentansprüche

1. Injektionsgerät mit einem Einstellteil (25), das sich beim Einstellen einer aus dem Injektionsgerät (1) auszupressenden Menge an Injektionsflüssigkeit gegenüber einem Gehäuse (2) des Injektionsgeräts (1) in einer ersten Drehrichtung (43) um eine Längsmittelachse (10) des Injektionsgeräts (1) dreht, und das sich beim Auspressen der Injektionsflüssigkeit aus dem Injektionsgerät (1) in einer der ersten Drehrichtung (43) entgegengerichteten zweiten Drehrichtung (44) dreht, wobei das Einstellteil (25) über eine erste Gewindeverbindung (26) mit dem Gehäuse (2) verbunden ist und sich beim Einstellen einer auszupressenden Menge an Injektionsflüssigkeit zusätzlich zur Drehung in der ersten Drehrichtung (43) in distaler Richtung bewegt und sich beim Auspressen einer eingestellten Menge an Injektionsflüssigkeit zusätzlich zur Drehung in der zweiten Drehrichtung (44) in proximaler Richtung bewegt, und mit einer Rasteinrichtung (72), die mindestens eine Raststellung des Einstellteils (25) definiert, wobei die Rasteinrichtung (72) zwischen dem Einstellteil (25) und dem Gehäuse (2) wirkt, und wobei die Rasteinrichtung (72) mindestens beim Einstellen der aus dem Injektionsgerät (1) auszupressenden Menge an Injektionsflüssigkeit wirksam ist, **dadurch gekennzeichnet, dass** jeder Raststellung eine eindeutige Drehlage des Einstellteils (25) gegenüber dem Gehäuse (2) zugeordnet ist.

2. Injektionsgerät nach Anspruch 1
**dadurch gekennzeichnet, dass** die mindestens eine Raststellung durch mindestens ein drehfest mit dem Gehäuse (2) verbundenes erstes Rastelement (30, 64, 65, 66, 67, 68) und mindestens ein drehfest mit dem Einstellteil (25) verbundenes, mit dem ersten Rastelement (30, 64, 65, 66, 67, 68) zusammenwirkendes zweites Rastelement (60) definiert ist.

3. Injektionsgerät nach Anspruch 2,
**dadurch gekennzeichnet, dass** die Rasteinrichtung (72) ein Rastteil (29) umfasst, das unabhängig vom Einstellteil (25) axial verschiebbar und drehfest mit dem Gehäuse (2) verbunden ist, und an dem mindestens ein erstes Rastelement (30, 64, 65, 66, 67, 68) angeordnet ist, wobei mindestens ein erstes Rastelement (30, 64, 65, 66, 67, 68) und mindestens ein zweites Rastelement (60) in einer ersten axialen Stellung von Rastteil (29) und Einstellteil (25) die mindestens eine Raststellung definieren und in mindestens einer zweiten axialen Stellung (49) von Rastteil (29) und Einstellteil (25) unabhängig von der relativen Drehlage des Einstellteils (25) zum Rastteil (29) außer Eingriff sind.

4. Injektionsgerät nach Anspruch 3,
**dadurch gekennzeichnet, dass** das Injektionsgerät (1) eine Feder (37) besitzt, die das Rastteil (29) in Richtung auf die erste axiale Stellung (48) vorspannt.

5. Injektionsgerät nach einem der Ansprüche 2 bis 4,
**dadurch gekennzeichnet, dass** mindestens ein Rastelement (30, 64, 65, 66, 67, 68) vor Erreichen einer Raststellung in Richtung der Längsmittelachse (10) des Injektionsgeräts (1) ausgelenkt wird.

6. Injektionsgerät nach einem der Ansprüche 1 bis 5,
**dadurch gekennzeichnet, dass** die Rasteinrichtung (72) in jeder Raststellung eine Relativdrehung des Einstellteils (25) gegenüber dem Gehäuse (2) in der ersten Drehrichtung (43) zulässt und in der zweiten Drehrichtung (44) sperrt.

7. Injektionsgerät nach einem der Ansprüche 1 bis 6,
**dadurch gekennzeichnet, dass** das Injektionsgerät (1) eine Feder (28) besitzt, die zwischen dem Einstellteil (25) und dem Gehäuse (2) wirkt und die das Einstellteil (25) in der zweiten Drehrichtung (44) vorspannt.

8. Injektionsgerät nach einem der Ansprüche 1 bis 7,
**dadurch gekennzeichnet, dass** das Injektionsgerät (1) eine Kupplung (33) besitzt, die in einer ersten Stellung (46) das Einstellteil (25) drehfest mit einem Bedienelement (6) verbindet und die in einer zweiten Stellung (47) eine Relativdrehung des Einstellteils (25) gegenüber dem Bedienelement (6) zulässt.

9. Injektionsgerät nach Anspruch 8,
**dadurch gekennzeichnet, dass** das Verstellen der Kupplung (33) von der ersten Stellung (46) in die zweite Stellung (47) durch Verschieben des Bedienelements (6) in proximaler Richtung erfolgt.

10. Injektionsgerät nach Anspruch 8 oder 9,
**dadurch gekennzeichnet, dass** das Rastteil (29) in axialer Richtung derart an das Bedienelement (6) gekoppelt ist, dass eine Bewegung des Bedienelements (6) in proximaler Richtung eine Bewegung des Rastteils (29) in proximaler Richtung bewirkt.

11. Injektionsgerät nach einem der Ansprüche 8 bis 10,
**dadurch gekennzeichnet, dass** das Rastteil (29) und das Bedienelement (6) axial fest und relativ zueinander drehbar miteinander verbunden sind.

12. Injektionsgerät nach einem der Ansprüche 8 bis 11,
**dadurch gekennzeichnet, dass** die Kupplung (33) eine erste Verzahnung (35) am Einstellteil (25) besitzt, die mit einer zweiten Verzahnung (34) am Bedienelement (6) zusammenwirkt.

13. Injektionsgerät nach einem der Ansprüche 8 bis 12,
**dadurch gekennzeichnet, dass** das Bedienelement (6) drehfest mit einem Zustellteil (24) verbunden ist, wobei das Zustellteil (24) über eine zweite Gewindeverbindung (27) mit einem Dosierkolben (12) verbunden ist, der drehfest gegenüber dem Gehäuse (2) gehalten ist.

14. Injektionsgerät nach Anspruch 13,
**dadurch gekennzeichnet, dass** das Einstellteil (25) derart auf das Zustellteil (24) wirkt, dass eine Bewegung des Einstellteils (25) in proximaler Richtung eine Bewegung des Zustellteils (24) in proximaler Richtung bewirkt.

## Claims

1. Injection device with an setting part (25) which, when setting a quantity of injection fluid to be expressed from the injection device (1), rotates relative to a housing (2) of the injection device (1) in a first direction of rotation (43) about a longitudinal central axis (10) of the injection device (1), and which, when expressing the injection fluid from the injection device (1), rotates in a second direction of rotation (44) opposed to the first direction of rotation (43), wherein the setting part (25) is connected to the housing (2) via a first threaded connection (26) and, when setting a quantity of injection fluid to be expressed, moves in a distal direction in addition to the rotation in the first direction of rotation (43) and, when expressing a set quantity of injection fluid, moves in a proximal direction in addition to the rotation in the second direction of rotation (44), and with a latching device (72), which defines at least one latching position of the setting part (25), wherein the latching device (72) acts between the setting part (25) and the housing (2), and wherein the latching device (72) is active at least when setting the quantity of injection fluid to be expressed from the injection device (1), **characterised in that** each latching position is assigned an unequivocal rotational position of the setting part (25) relative to the housing (2).

2. Injection device according to claim 1,
**characterised in that** the at least one latching position is defined by at least one first latching element (30, 64, 65, 66, 67, 68) non-rotatably connected to the housing (2) and at least one second latching element (60) non-rotatably connected to the setting part (25) and acting together with the first latching element (30, 64, 65, 66, 67, 68).

3. Injection device according to claim 2,
**characterised in that** the latching device (72) comprises a latching part (29), which is axially displaceable independently of the setting part (25) and non-rotatably connected to the housing (2), and on which at least one first latching element (30, 64, 65, 66, 67, 68) is located, wherein at least one first latching element (30, 64, 65, 66, 67, 68) and at least one second latching element (60) define the at least one latching position in a first axial position of the latching part (29) and the setting part (25) and are out of engagement with the latching part (29) in a second axial position (49) of the latching part (29) and the setting part (25) independently of the relative rotary position of the setting part (25) and the latching part (29).

4. Injection device according to claim 3,
**characterised in that** the injection device (1) has a spring (37), which preloads the latching part (29) towards the first axial position (48).

5. Injection device according to any of claims 2 to 4,
**characterised in that** at least one latching element (30, 64, 65, 66, 67, 68) is deflected towards the longitudinal central axis (10) of the injection device (1) before reaching a latching position.

6. Injection device according to any of claims 1 to 5,
**characterised in that** the latching device (72) allows a rotation of the setting part (25) relative to the housing (2) in the first direction of rotation (43) in each latching position and blocks it in the second direction of rotation (44).

7. Injection device according to any of claims 1 to 6,
**characterised in that** the injection device (1) has a spring (28), which acts between the setting part (25) and the housing (2) and which preloads the setting part (25) in the second direction of rotation (44).

8. Injection device according to any of claims 1 to 7,
**characterised in that** the injection device (1) has a clutch (33), which connects the setting part (25) non-rotatably to a control (6) in a first position (46) and allows a rotation of the setting part (25) relative to the control (6) in a second position (47).

9. Injection device according to claim 8,
**characterised in that** the clutch (33) is moved from the first position (46) to the second position (47) by shifting the control (6) in the proximal direction.

10. Injection device according to claim 8 or 9,
**characterised in that** the latching part (29) is coupled to the control (6) in the axial direction in such a way that a movement of the control (6) in the proximal direction causes a movement of the latching part (29) in the proximal direction.

11. Injection device according to any of claims 8 to 10,
**characterised in that** the latching part (29) and the control (6) are connected to each other in an axially fixed manner while being rotatable relative to each other.

12. Injection device according to any of claims 8 to 11,
**characterised in that** the clutch (33) has a first toothing (35) at the setting part (25), which acts together with a second toothing (34) at the control (6).

13. Injection device according to any of claims 8 to 12,
**characterised in that** the control (6) is non-rotatably connected to a feed part (24), the feed part (24) being connected via a second threaded connection (27) to a dosing piston (12), which is held non-rotatably relative to the housing (2).

14. Injection device according to claim 13,
**characterised in that** the setting part (25) acts on the feed part (24) in such a way that a movement of the setting part (25) in the proximal direction causes a movement of the feed part (24) in the proximal direction.

## Revendications

1. Dispositif d'injection avec une partie de réglage (25), qui tourne lors du réglage d'une quantité de liquide d'injection à éjecter hors du dispositif d'injection (1) par rapport à un boîtier (2) du dispositif d'injection (1) dans un premier sens de rotation (43) autour d'un axe médian longitudinal (10) du dispositif d'injection (1), et qui tourne lors de l'éjection du liquide d'injection hors du dispositif d'injection (1) dans un deuxième sens de rotation (44) opposé au premier sens de rotation (43), dans lequel la partie de réglage (25) est reliée au boîtier (2) par le biais d'une première liaison filetée (26) et se déplace lors du réglage d'une quantité de liquide d'injection à éjecter en plus de la rotation dans le premier sens de rotation (43) dans la direction distale et se déplace lors de l'éjection d'une quantité réglée de liquide d'injection en plus de la rotation dans le deuxième sens de rotation (44) dans la direction proximale, et avec un dispositif d'arrêt (72), qui définit au moins une position d'arrêt de la partie de réglage (25), dans lequel le dispositif d'arrêt (72) agit entre la partie de réglage (25) et le boîtier (2), et dans lequel le dispositif d'arrêt (72) est actif au moins lors du réglage de la quantité de liquide d'injection à éjecter hors du dispositif d'injection (1), **caractérisé en ce qu'**une position de rotation univoque de la partie de réglage (25) par rapport au boîtier (2) est associée à chaque position d'arrêt.

2. Dispositif d'injection selon la revendication 1,
**caractérisé en ce que** l'au moins une position d'arrêt est définie par au moins un premier élément d'arrêt (30, 64, 65, 66, 67, 68) relié solidaire en rotation au boîtier (2) et au moins un deuxième élément d'arrêt (60) coopérant avec le premier élément d'arrêt (30, 64, 65, 66, 67, 68), relié solidaire en rotation à la partie de réglage (25).

3. Dispositif d'injection selon la revendication 2,
**caractérisé en ce que** le dispositif d'arrêt (72) comprend une partie d'arrêt (29), qui est reliée indépendamment de la partie de réglage (25) de manière axialement coulissante et solidaire en rotation au boîtier (2), et au niveau de laquelle au moins un premier élément d'arrêt (30, 64, 65, 66, 67, 68) est agencé, dans lequel au moins un premier élément d'arrêt (30, 64, 65, 66, 67, 68) et au moins un deuxième élément d'arrêt (60) dans une première position axiale de la partie d'arrêt (29) et partie de réglage (25) définissent l'au moins une position d'arrêt et sont hors prise dans au moins une deuxième position axiale (49) de la partie d'arrêt (29) et partie de réglage (25) indépendamment de la position de rotation relative de la partie de réglage (25) par rapport à la partie d'arrêt (29).

4. Dispositif d'injection selon la revendication 3,
**caractérisé en ce que** le dispositif d'injection (1) dispose d'un ressort (37), qui précontraint la partie d'arrêt (29) en direction de la première position axiale (48).

5. Dispositif d'injection selon l'une quelconque des revendications 2 à 4,
**caractérisé en ce qu'**au moins un élément d'arrêt (30, 64, 65, 66, 67, 68) est dévié avant l'atteinte d'une position d'arrêt en direction de l'axe médian longitudinal (10) du dispositif d'injection (1).

6. Dispositif d'injection selon l'une quelconque des revendications 1 à 5,
**caractérisé en ce que** le dispositif d'arrêt (72) autorise dans chaque position d'arrêt une rotation relative de la partie de réglage (25) par rapport au boîtier (2) dans le premier sens de rotation (43) et la bloque dans le deuxième sens de rotation (44).

7. Dispositif d'injection selon l'une quelconque des revendications 1 à 6,
**caractérisé en ce que** le dispositif d'injection (1) dispose d'un ressort (28), qui agit entre la partie de réglage (25) et le boîtier (2) et qui précontraint la partie de réglage (25) dans le deuxième sens de rotation (44).

8. Dispositif d'injection selon l'une quelconque des revendications 1 à 7,
**caractérisé en ce que** le dispositif d'injection (1) dispose d'un embrayage (33), qui relie dans une première position (46) la partie de réglage (25) de manière solidaire en rotation à un élément de commande (6) et qui autorise dans une deuxième position (47) une rotation relative de la partie de réglage (25) par rapport à l'élément de commande (6).

9. Dispositif d'injection selon la revendication 8,
**caractérisé en ce que** le réglage de l'embrayage (33) de la première position (46) à la deuxième position (47) a lieu par coulissement de l'élément de commande (6) dans la direction proximale.

10. Dispositif d'injection selon la revendication 8 ou 9,
**caractérisé en ce que** la partie d'arrêt (29) est couplée dans la direction axiale au niveau de l'élément de commande (6) de sorte qu'un déplacement de l'élément de commande (6) dans la direction proximale entraîne un déplacement de l'élément d'arrêt (29) dans la direction proximale.

11. Dispositif d'injection selon l'une quelconque des revendications 8 à 10,
**caractérisé en ce que** la partie d'arrêt (29) et l'élément de commande (6) sont reliés l'un à l'autre de manière axialement fixe et rotative l'un par rapport à l'autre.

12. Dispositif d'injection selon l'une quelconque des revendications 8 à 11,
**caractérisé en ce que** l'embrayage (33) dispose d'une première denture (35) au niveau de la partie de réglage (25), qui coopère avec une deuxième denture (34) au niveau de l'élément de commande (6).

13. Dispositif d'injection selon l'une quelconque des revendications 8 à 12,
**caractérisé en ce que** l'élément de commande (6) est relié solidaire en rotation à une partie de distribution (24), dans lequel la partie de distribution (24) est reliée à un piston de dosage (12) par le biais d'une deuxième liaison filetée (27), qui est retenu solidaire en rotation par rapport au boîtier (2).

14. Dispositif d'injection selon la revendication 13,
**caractérisé en ce que** la partie de réglage (25) agit sur la partie de distribution (24) de sorte qu'un déplacement de la partie de réglage (25) dans la direction proximale entraîne un déplacement de la partie de distribution (24) dans la direction proximale.
